(19) Europäisches Patentamt
European Patent Office
Office européen des brevets

(11) Veröffentlichungsnummer : **0 456 917 B1**

(12) # EUROPÄISCHE PATENTSCHRIFT

(45) Veröffentlichungstag der Patentschrift :
**01.09.93 Patentblatt 93/35**

(51) Int. Cl.$^5$ : **A61L 15/07**

(21) Anmeldenummer : **90203538.5**

(22) Anmeldetag : **13.12.90**

(54) **Orthopädisches Verbandmaterial mit verringerter Klebrigkeit und vermindertem Rutschverhalten.**

(30) Priorität : **18.12.89 US 452217**

(43) Veröffentlichungstag der Anmeldung :
**21.11.91 Patentblatt 91/47**

(45) Bekanntmachung des Hinweises auf die Patenterteilung :
**01.09.93 Patentblatt 93/35**

(84) Benannte Vertragsstaaten :
**AT BE CH DE DK ES FR GB GR IT LI LU NL SE**

(56) Entgegenhaltungen :
**EP-A- 0 221 669**
**EP-A- 0 295 031**
**EP-A- 0 305 804**
**US-A- 4 667 661**

(73) Patentinhaber : **Lohmann GmbH & Co. KG**
**Irlicher Strasse 55**
**D-56567 Neuwied (DE)**

(72) Erfinder : **Edenbaum,Martin**
**12 Springwood Drive**
**Princeton Junction, New Jersey 08650 (US)**
Erfinder : **Frisch,Kurt Charles**
**17988 Park Lane**
**Goss Ile, Michigan 48138 (US)**
Erfinder : **Sendijarevic,Alsa**
**1168 Woodside Trail**
**Troy, Michigan 48098 (US)**
Erfinder : **Wong,Shalo-won**
**22518 Rio Vista**
**St.Claire Shores, Michigan 48081 (US)**

(74) Vertreter : **Flaccus, Rolf-Dieter, Dr.**
**Patentanwalt Sperlingsweg 32**
**D-50389 Wesseling (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft einen orthopädischen Steifverband, enthaltend ein textiles Flächengebilde, das mit einer Kombination aus einem reaktiven, fluiden Polyisocyanatprepolymeren beschichtet oder imprägniert ist, das bei Befeuchtung des Harzes mit Wasser unter Aushärtung zu Polyurethanen reagiert und zur Reduzierung von Klebrigkeit, Rutschen und Schäumen wenigstens einen Hilfsstoff enthält.

Die meisten heute zur Verfügung stehenden orthopädischen Steifverbände werden unter Verwendung aushärtbarer Harze hergestellt, die auf ein Substrat wie Glasfaser, Polyester oder andere synthetische oder natürliche textile Flächengebilde aufgebracht werden. So sind z.B. orthopädische Steifverbände mit Polyisocyanatprepolymeren, die mit Wasser reagieren und so die Aushärtung zu Polyurethanen initiieren, bereits bekannt (US-PS 4,411,262 von Bonin et al, US-PS 4,502,479 Garwood et al). Im allgemeinen umfaßt das Polyisocyanatprepolymer das Reaktionsprodukt eines Isocyanats und eines Polyols, welches bei Kontakt mit Wasser zu Polyurethan polymerisiert. Die mit Prepolymer behandelte Binde wird vor dem Aufbringen auf ein Körperteil mit Wasser getränkt, und die nasse Binde wird dann auf den Körperteil aufgebracht. Nach Aufbringen der Binde wird der Steifverband mit einem Handschuh geglättet und an bestimmten Punkten festgehalten, bis er hart wird. Da die Harze in dem Verband bis zu ihrer Aushärtung sehr klebrig sind, bleiben die Schutzhandschuhe der Person, die den Verband anbringt, oftmals am Verband kleben. Dies ist von Nachteil, da es zum Aufwickeln des Verbandes kommen kann, weil Schichten des Verbandes sich voneinander trennen, und der Verband somit nicht mehr geformt werden kann.

Um die "Klebrigkeit" aushärtbarer, harzbeschichteter Verbände zu verringern, wurde von Scholz et al in US-PS 4,667,661 vorgeschlagen, solche Verbandmaterialien mit bestimmten Schmiermitteln zu behandeln, um den kinetischen Reibungskoeffizienten solcher Flächen auf weniger als etwa 1,2 zu reduzieren. Dieses Gleitmittel kann aus (a) hydrophilen Gruppen, die kovalent an das aushärtbare Harz gebunden sind, (b) einem Hilfsstoff, der mit dem aushärtbaren Harz inkompatibel ist oder (c) einer Kombination aus (a) und (b) bestehen. Wie Scholz et al weiter ausführen (z.B. Spalte 11, Zeilen 21 ff.), werden mit solchen Gleitmitteln behandelte Verbände sehr rutschig, und das Verformen des Verbandes wird wegen der nichtklebrigen Eigenschaften des Harzes erleichert. Im gleichen Patent (Spalte 8, Zeilen 45 bis 65) wird ferner angeführt, daß Materialien wie Mineralöl als Schmiermittel beurteilt wurden, und, obwohl sie der Oberfläche des Steifverbandes eine nichtklebrige und sogar rutschige Eigenschaft verliehen, die einfache Anbringung und Formbarkeit der Binde am Patienten erlaubt, diese Wirkung nur vorübergehender Natur war. Im Durchschnitt hielten diese Materialien nur von einem Tag bis zu einer Woche, augenscheinlich wegen der Auflösung des Öls in dem Harz.

Es besteht daher ein Bedarf an Verbandmaterialien mit verbesserten Handhabungseigenschaften, d.h. die weder zu klebrig noch zu rutschig sind.

Es wurde nun gefunden, daß solche Verbandmaterialien erhalten werden können, indem ein hydrophiles Bisurethan in das zur Beschichtung der Verbandmaterialien benutzte Polyisocyanatprepolymer gemischt wird. Insbesondere betrifft die vorliegende Erfindung eine Prepolymermischung für den Einsatz in orthopädischen Steifverbänden, wobei diese Mischung ein Polyisocyanatprepolymer und eine wirksame "antiklebrigmachende" Menge eines oder mehrerer hydrophiler Bisurethane enthält. Die vorliegende Erfindung betrifft ebenso orthopädische Verbandmaterialien, die ein textiles Flächengebilde, das mit dieser Prepolymermischung imprägniert und/oder beschichtet ist, enthalten.

Hydrophile Bisurethane, die als antiklebrigmachende Hilfsstoffe gemäß der vorliegenden Erfindung eingesetzt werden können, sind Verbindungen, die aus den Formeln I und II ausgewählt sind:

$$RO(O)CNH\text{-}X\text{-}NHC(O)O\text{-}Y\text{-}O(O)CNH\text{-}X\text{-}NHC(O)R \qquad (I)$$

$$Z\text{-}NHC(O)O\text{-}Y\text{-}O(O)CNH\text{-}Z \qquad (II)$$

worin Y eine hydrophile Polymerkette mit einem Molekulargewicht in der Größenordnung von etwa 1000 bis 8000 ist. Y kann von Dilolen der allgemeinen Formel (III):

$$H(OCH_2CH_2)_m - (O\overset{CH_3}{\overset{|}{C}}HCH_2)_n - OR'O - (CH_2\overset{CH_3}{\overset{|}{C}}HO)_n - (CH_2CHO)_m\ H$$

(III)

abgeleitet sein, worin m = eine ganze Zahl von 1 oder mehr, n = 0 oder eine ganze Zahl von 1 oder mehr und R' = $-CHCH_2-$ oder $-CH(CH_3)CH_2-$ ist. Wenn n gleich 0 ist, ist die Verbindung nach Formel III ein Poly(oxyethylen)diol. Das Polymer kann ein Blockcopolymer oder ein statistisches Copolymer sein.

Jedes X der Formel I und jedes Z der Formel II kann gleich oder verschieden sein und ist ausgewählt aus einer aromatischen, cycloaliphatischen oder aliphatischen Gruppe. Bei der Formel I sind die Bisurethane aus

den oben beschriebenen Polyetherdiolen (III) und Diisocyanaten abgeleitet, die sowohl aromatisch (z.B. Toluoldiisocyanat, Methylen-4,4'-bis(phenylisocyanat), cycloaliphatisch (z.B. Methylen-4,4'-bis(cyclohexyl) diisocyanat, Isophorondiisocyanat, 1,4-Cyclohexandiisocyanat), aliphatisch (z.B. Hexamethylendiisocyanat) als auch Mischungen daraus sein können. Bei der Formel II sind die Bisurethane aus den oben beschriebenen Polyetherdiolen (III) und Monoisocyanaten abgeleitet, die aromatisch (z.B. Phenylisocyanat), cycloaliphatisch (z.B. Cyclohexylisocyanat), aliphatisch (z.B. Butylisocyanat) oder auch Mischungen daraus sein können.

In Formel I steht R für -OR", wobei -OR" von monofunktionellen Alkoholen, wie z.B. Methanol, Ethanol, Isopropanol, Butanol, Laurylalkohol u.a., oder von Oxyalkylenaddukten monofunktioneller Alkohole der allgemeinen Formel IV:

$$R''' - O - (CH_2\overset{CH_3}{\overset{|}{C}}HO)_q(CH_2CH_2)_mH$$

(IV)

abgeleitet ist, worin R''' H oder Alkyl ist und q und m unabhängig 0 oder ganze Zahlen von 1 oder mehr sind, vorausgesetzt, daß, wenn entweder q oder m 0 ist, der jeweils andere eine ganze Zahl von 1 oder mehr ist. Die besten Ergebnisse wurden mit Bisurethanen der Formel I erreicht, worin R von Methanol oder Ethanol abgeleitet ist.

Um die Bisurethane der Formel I herzustellen, wird ein Mol Polyetherdiol mit zwei Molen Diisocyanat umgesetzt, um ein auf Isocyanat endendes Prepolymer herzustellen, welches dann mit zwei Molen monofunktionellem Alkohol umgesetzt wird. Um die Bisurethane der Formel II herzustellen, wird ein Mol eines Polyetherdiols der allgemeinen Formel III mit zwei Molen monofunktionellem Isocyanat umgesetzt. Die Synthese der Bisurethane kann im Lösungsmittel oder in der Masse durchgeführt werden. Es können unterschiedliche nicht-reaktive Lösungsmittel verwendet werden, so z.B. Cellosolveacetat und Dichlormethan. Bei der Bisurethanherstellung ist die Reaktionstemperatur kritisch und sollte 80°C nicht übersteigen, da ansonsten Nebenreaktionen auftreten können. Die Synthese sollte unter inerten, trockenen Bedingungen mit getrockneten Reagenzien durchgeführt werden. Zur Steuerung der Reaktionsrate können bei der Herstellung der Bisurethane kleine Mengen Benzoylchlorid (0,5-1%) eingesetzt werden.

Erfindungsgemäß werden die Bisurethane der Formeln I und II bevorzugt, worin Y aus der Gruppe bestehend aus Polyethylenoxiden, Polypropylenoxiden und regellosen Copolymeren oder Blockcopolymeren von Ethylen-/Propylenoxid ausgewählt ist. Die am meisten bevorzugte hydrophile Gruppe ist ein Polyethylenglykol mit einem Molekulargewicht in der Größenordnung von etwa 3000 bis 5000, insbesondere bevorzugt etwa 4000.

Das erfindungsgemäß verwendete Polyisocyanatprepolymer enthält ein Prepolymer, das von Polyisocyanat abgeleitet und vorzugsweise aromatisch ist, und eine reaktive Wasserstoffverbindung oder ein Oligomer. Die bevorzugte Prepolymerzusammensetzung enthält modifiziertes Diphenylmethandiisocyanat, Polypropylenglykol, Benzoylchloridstabilisator und Dimorpholindiethyletherkatalysator. Das bevorzugte Polyisocyanat-Diolverhältnis beträgt etwa 4 zu 1 (NCO/OH = 4/1). Zur Verlängerung der Lagerbeständigkeit des Materials kann das Prepolymer bestimmte Stabilisatoren, wie Benzoylchlorid (0,1 bis 1,0 Gew.-%) enthalten, ebenso können Schaumdämpfungsmittel, wie Silikonflüssigkeiten enthalten sein.

Die vorteilhaften nichtklebrigen und doch nichtrutschigen Eigenschaften des erfindungsgemäßen Steifverbandes werden erreicht, indem mit dem Polyisocyanatprepolymer eine wirksame antiklebrigmachende Menge eines oben beschriebenen hydrophilen Bisurethans vermischt wird. Im allgemeinen sollte das hydrophile Bisurethan mit einem Polyisocyanatprepolymeren in einer Menge von etwa 0,1 bis 10%, vorzugsweise 0,5 bis 5%, und besonders bevorzugt von etwa 2% pro Gewichtseinheit des Polyisocyanatprepolymeren gemischt werden.

Erfindungsgemäß wurden die besten Ergebnisse mit einem Polyisocyanatprepolymeren erzielt, der das Reaktionsprodukt darstellt aus 56% Diphenylmethandiisocyanat, 37,7% Polypropylenglykol (Pluracol P710, BASF Chemicals), 0,1% Benzoylchlorid, 2,0% Dimorpholinyldiethyletherkatalysator, 0,2% Silikonschaumdämpfungsmittel und 4% einer antiklebrigmachenden Mischung hergestellt durch Kontakt von 11% 4,4'-Diphenylmethandiisocyanat, 0,5% Benzoylchlorid und 8,7% Poly(oxyethylen)glykol (Molekulargewicht = 4000), wobei die Reaktion kurz zwecks Zugabe von 1,5% Ethanol unterbrochen wurde (alle hier und in der weiteren Beschreibung benutzten Prozentangaben beziehen sich, sofern nichts Gegenteiliges angeführt ist, auf Gewichtsprozent der gesamten Reaktionsprodukte).

Textile Flächengebilde, die mit einem aushärtbaren Polyisocyanatprepolymer beschichtet werden oder in die ein solches Prepolymer imprägniert werden kann, sind im Stand der Technik ausführlich beschrieben (z.B.

US-PS 4,667,661 und US-PS 4,411,262, deren Beschreibungen durch Bezugnahme in die vorliegende Anmeldung aufgenommen werden). Die Bahn ist halbstarr oder flexibel und sollte porös sein, so daß der Härter, Wasser, in die Rolle des textilen Flächenmaterials eindringen kann und alle Teile des Harzes berührt. Geeignete Bahnen sind z.B. Gewebe, Vliesstoffe oder Gewirke aus natürlichen oder synthetischen Fasern. Vorzugsweise sind die Bahnen Glasfasergewirke, es können jedoch auch textile Flächengebilde aus z.B. Baumwolle und Polyester eingesetzt werden.

Die auf das textile Flächengebilde aufgetragene Prepolymer/Antiklebrigmachermischung muß ausreichen, um eine starke Laminatverbindung zwischen den Schichten zu bilden, darf jedoch nicht so groß sein, daß die Porosität verloren geht und der Harzfilm, der zur raschen und vollständigen Aushärtung möglichst dünn sein sollte, unnötig dick wird. Überschüssiges Prepolymer kann zu unsauberer Handhabung des textilen Flächengebildes aufgrund von Klebrigkeit oder abtropfender Flüssigkeit und Übertragung von Harz führen. Das gewünschte Gewichtsverhältnis Harz/Trägermaterial ist eine Funktion sowohl der Viskosität des Prepolymeren als auch der Oberflächeneigenschaften des textilen Flächengebildes und somit nur schwerlich genau quantifizierbar, der Fachmann wird jedoch ein geeignetes Verhältnis leicht bestimmen können.

Die Materialien gemäß der vorliegenden Erfindung werden in den nachfolgenden Beispielen, die lediglich der Verdeutlichung dienen ohne die Erfindung jedoch einzuschränken, näher erläutert.

Beispiel 1

Herstellung von auf NCO-endenden-Urethanprepolymeren

Die in Tabelle I aufgeführten auf NCO-endenden Urethanprepolymeren wurden nach dem folgenden allgemeinen Verfahren hergestellt.

Die eingesetzten Polyole wurden vor Gebrauch bei 80°C im Vakuum von 103 mm Hg 24 Stunden getrocknet.

Durch Mischen von zwei (2) Äquivalenten von 4,4'-Diphenylmethandiisocyanat ("MDI", Mondur M, Mobay Chemical Co.) mit einem (1) Äquivalent Polyol wurde ein auf NCO endendes Urethanprepolymer hergestellt. Die MDI-Flocken wurden in einen 500 ml-Reaktionskessel eingewogen, der mit Tropftrichter, Stickstoffeinlaß- und -auslaßventil, mechanischem Rührer, Heizmantel und Thermometer ausgerüstet war. Das MDI wurde bis zum Schmelzen bei 70°C unter einer trockenen Stickstoff-Decke erhitzt. 0,5% Benzoylchlorid, bezogen auf die Gesamtformulierung (MDI + Polyol), wurde zu dem geschmolzenen MDI gegeben. Das Benzoylchlorid vermischte sich mit dem MDI bei 70°C bis die Mischung homogen wurde. Die errechnete Menge an Polyol wurde zu der gerührten MDI-Benzoylchloridmischung bei 70°C im gleichmäßigen Strom zugegeben. Die Reaktionstemperatur wurde je nach Menge und Rate, bei der das Polyol zugegeben wurde, auf über 80°C erhöht. Die Reaktion wurde über Wasserbad gesteuert. Im Anschluß an die Polyolzugabe, sollte die Temperatur bei 70°C gehalten werden. Die Reaktion war innerhalb von 3 bis 4 Stunden abgeschlossen.

Nach dem Reaktionsablauf wurde die n-Dibutylamintitration (ASTM D-1638-84) durchgeführt. Die Reaktion wurde als abgeschlossen angesehen, als der festgelegte Prozentwert an Isocyanat im Prepolymeren im Rahmen von 1% dem theoretischen Wert, der aufgrund der Gewichte der eingesetzten Materialien errechnet wurde, entsprach.

Tabelle I

Zusammensetzung und Konsistenz der Bisurethane

| Beisp. | Komponente | | | Konsistenz |
|---|---|---|---|---|
| | Polyol (mol) | Monofunkt. Alkohol (mol) | Isocyanat (mol) | |
| 1 | PEG-1000<br>1 | EtOH<br>2 | MDI<br>2 | Fest |
| 2 | PEG-1500<br>1 | EtOH<br>2 | MDI<br>2 | Fest |
| 3 | PEG-4000<br>1 | EtOH<br>2 | MDI<br>2 | Fest |
| 4 | PEG-8000<br>1 | EtOH<br>2 | MDI<br>2 | Fest |
| 5 | PEG-4000<br>1 | MeOH<br>2 | MDI<br>2 | Fest |
| 6 | PEG-4000<br>1 | EtOH<br>2 | IPDI<br>2 | Unlösliches Gel |
| 7 | PEG-4000<br>1 | - | PI<br>2 | Fest |
| 8 | PEG-4000<br>1 | - | PI<br>2 | Fest |
| 9 | - | Polyoxy-ethylen-derivat von Nonylphenol MG 632 | MDI<br>1 | Flüssig |

PEG-1000 = Poly-G-1000, Olin Chemical
PEG-1500 = Poly-G-1500, Olin Chemical
PEG-4000 = Pluracol E-4000, BASF Chemicals
PEG-8000 = Pluracol E-8000, BASF Chemicals
Polyoxyethylenderivat von Nonylphenol = Surfonic N-95, Texaco Chemicals
MDI = 4,4'-Diphenylmethandiisocyanat, Mondur M, Mobay Chemicals
IPDI = Isophorondiisocyanat, "IPDI", Huels America
PI = Phenylisocyanat, "PI", Aldrich Chemicals

Herstellung von Bisurethan

Zu dem gerührten Urethanprepolymeren wurde eine äquivalente Menge 100%iges Ethanol bei 70°C unter Stickstoff in einem Überschuß von 2-3 S zugegeben. Der Kessel wurde zur Verhinderung der Alkoholverdampfung mit einem Wasserkühler ausgestattet. Durch einen Tropftrichter wurde der Alkohol bei Raumtemperatur

in einem Teil zu dem gerührten Prepolymeren gegeben. Die Reaktion zwischem dem NCO-Prepolymeren und dem Alkohol trat sehr schnell bei 70°C ein; sie war innerhalb von 2 Stunden abgeschlossen. Der Reaktion folgte wiederum die n-Dibutylamintitration, sie wurde als beendet angesehen, als der Prozentanteil Isocyanat im Material Null betrug. Das Bisurethanmaterial wurde sodann in eine geschlossene Glasflasche bzw. einen Metallbehälter übertragen und dort gelagert.

Die nach dem oben beschriebenen Verfahren hergestellten Bisurethane wurden bei Raumtemperatur mit Polyisocyanatprepolymer (Reaktionsprodukt aus Diphenylmethandiisocyanat und Polypropylenglykol) gemischt. Die bei Raumtemperatur festen Bisurethane wurden vor dem Mischen bei 70 bis 80°C geschmolzen. Nach 24-stündigem Verweilen bei Raumtemperatur wurden die entstandenen Mischungen auf Kompatibilität, Viskosität, Isocyanatkonzentration, Klebrigkeit und Rutschverhalten geprüft.

Die Klebrigkeit und das Rutschverhalten wurden nach dem folgenden Verfahren gemessen: Ein Streifen oder Band wird mit der Polyisocyanatprepolymer/Bisurethanmischung beschichtet. Das beschichtete Band wird 5 mal in Wasser getaucht. Die Rutscheigenschaften werden durch Aufzeichnen der Rutschigkeit von Gummihandschuhen auf der beschichteten Oberfläche gemessen.

Die Prüfmethode zur Bestimmung der Klebrigkeit ist eine qualitative Prüfung. Die Harzmischung wird auf ca. 10,20 cm (4 inches) eines 2,54 x 12,7 cm ( 1" x 5") langen Bandes aufgetragen, wobei 2,54 cm (1 inch) des Bandes trocken gelassen wurden. Das beschichtete Band wird in Wasser getaucht, flach gelegt, und ein 2,54 x 10,16 cm (1" x 4") breites Stück eines Gummihandschuhs wird fest darauf angedrückt. 2,54 cm (1 inch) des unbeschichteten Teiles des Bandes wird mit einer Wäscheklammer an dem ringförmigen Teil eines Stativs befestigt. Anschließend wird ein an einer Schnur hängendes 5-g-Gewicht an dem Band befestigt; dazu wird eine Büroklammer benutzt, die an dem mit dem Gummihandschuh versehenen Teil ca. 0,32 cm (1/8 inch) vom Rand entfernt angebracht wird. Das losgelassene Gewicht soll das Gummi von dem Band abziehen. Die Zeit bis zur vollständigen Ablösung des 10,16 cm (4 inches) breiten Gummistreifens von dem Band wird mit einer Stoppuhr gemessen.

Die Eigenschaften der erhaltenen Gießharze sind in Tabelle II aufgeführt.

Tabelle II

| Beisp. | Konzentr. an Bisurethan (%) | Viskosität cps | Ablösezeit sek. | % NCO |
|---|---|---|---|---|
| Kontrolle | 0 | 13.400 | 15 | 11,27 |
| 1 | 2 | 12.800 | 8 | 9,60 |
| 2 | 2 | 11.400 | 5 | 10,31 |
| 3 | 2 | 17,400 | 6 | 10,33 |
| 4 | 2 | 102.600 | sehr lang | 9,92 |
| 5 | 2 | 23.400 | 12 | 10,02 |
| 6 | - | - | - | - |
| 7 | 2 | 13.400 | 3,5 | 10,78 |
| 8 | 2 | 20.600 | 2,2 | 8,59 |
| 9 | 2 | 22.200 | 7.0 | 8,75 |

**Patentansprüche**

1. Orthopädischer Steifverband, enthaltend ein textiles Flächengebilde, das mit einer Kombination aus einem reaktiven, fluiden Polyisocyanatprepolymeren beschichtet oder imprägniert ist, das bei Befeuchtung des Harzes mit Wasser unter Aushärtung zu Polyurethanen reagiert und zur Reduzierung von Klebrigkeit, Rutschen und Schäumen wenigstens einen Hilfsstoff enthält, dadurch gekennzeichnet, daß in das zur Beschichtung des Flächengebildes verwendete Polyisocyanatprepolymer als antiklebrigmachende Hilfsstoffe hydrophile Bisurethane in einer Menge von etwa 0,5 bis 5 Gew.-%, bezogen auf das Gewicht des

Prepolymeren, eingesetzt sind, deren Verbindungen aus den Formeln I und II ausgewählt sind :

R(O)CNH-X-NHC(O)O-Y-O(O)CNH-X-NHC(O)R (I)

Z-NHC(O)O-Y-O(O)CNH-Z (II)

worin Y eine hydrophile Polymerkette mit einem Molekulargewicht in der Größenordnung von etwa 1000 bis 8000 ist, jedes X in Formel I und jedes Z in Formel II gleich oder verschieden sein kann und ausgewählt ist aus aromatischen, cycloaliphatischen oder aliphatischen Gruppen, R -OR" ist, wobei -OR" von monofunktionellen C1-C16 Alkoholen oder von Oxyalkylenaddukten monofunktioneller Alkohole der allgemeinen Formel IV:

$$R''' - O - (CH_2\overset{\overset{CH_3}{|}}{C}HO)_q(CH_2CH_2)_mH$$

(IV)

abgeleitet ist, worin R' " H oder Alkyl ist und q und m unabhängig 0 oder ganze Zahlen von 1 oder mehr sind, unter der Bedingung, daß, wenn entweder q oder m 0 ist, der jeweils andere eine ganze Zahl von 1 oder mehr ist.

2. Steifverband gemäß Anspruch 1, dadurch gekennzeichnet, daß das textile Flächengebilde ein Glasfasergewirke ist.

3. Steifverband gemäß Anspruch 1, dadurch gekennzeichnet, daß das Polyisocyanatprepolymer das Reaktionsprodukt aus Diphenylmethandiisocyanat und Polypropylenglykol enthält.

4. Steifverband nach Anspruch 1, dadurch gekennzeichnet, daß das Y von Diolen der allgemeinen Formel (III):

$$H(OCH_2CH_2)_m - (O\overset{\overset{CH_3}{|}}{C}HCH_2)_n - OR'O - (CH_2\overset{\overset{CH_3}{|}}{C}HO)_n - (CH_2CHO)_mH$$

(III)

abgeleitet ist, worin m eine ganze Zahl von 1 oder mehr ist und n = 0 oder eine ganze Zahl von 1 oder mehr, und R' = $-CHCH_2-$ oder $-CH(CH_3)CH_2-$ ist.

5. Steifverband nach Anspruch 4, dadurch gekennzeichnet, daß Y aus der Gruppe bestehen aus Polyethylenoxiden, Polypropylenoxiden und regellosen oder Block-Copolymeren von Ethylen-/Propylenoxid ausgewählt ist.

6. Steifverband nach Anspruch 5, dadurch gekennzeichnet, das Y ein Polyethyleglycol mit einem Molekulargewicht in der Größenordnung von etwa 3000 bis 5000 ist.

7. Steifverband nach Anspruch 6, dadurch gekennzeichnet, daß das Y ein Polyethylenglykol mit einem Molekulargewicht von etwa 4000 ist.

8. Steifverband nach Anspruch 1, dadurch gekennzeichnet, daß das Bisurethan die Formel I aufweist.

9. Steifverband nach Anspruch 1, dadurch gekennzeichnet, daß das Bisurethan die Formel II aufweist.

10. Steifverband nach Anspruch 8, dadurch gekennzeichnet, daß das X von einem Diisocyanat abgeleitet ist, welches aus der Gruppe bestehend aus Toluoldiisocyanat, Methylen-4,4'--bis(phenylisocyanat), Methylen-4,4'-bis(cylohexyl)diisocyanat, Isophorondiisocyanat, 1,4-cyclohexandiisocyanat, Hexamethylendiisocyanat und Mischungen daraus ausgewählt ist.

11. Steifverband nach Anspruch 9, dadurch gekennzeichnet, daß das X von einem Monoisocyanat abgeleitet ist, welches aus der Gruppe bestehend aus Phenylisocyanat, Cyclohexylisocyanat, Butylisocyanat und

Mischungen daraus ausgewählt ist.

**12.** Steifverband nach Anspruch 8, dadurch gekennzeichnet, daß -OR" von Methanol oder Ethanol abgeleitet ist.

**13.** Steifverband nach Anspruch 8, dadurch gekennzeichnet, daß das Y ein Polyethylenglykol mit einem Molekulargewicht in der Größenordnung von etwa 3000 bis 5000 ist, X von Methylen -4,4'-bis(phenylisocyanat) und -OR" von Ethanol abgeleitet ist.

**14.** Steifverband nach Anspruch 13, dadurch gekennzeichnet, daß Y ein Molekulargewicht von etwa 4000 aufweist.

**15.** Steifverband nach Anspruch 1, dadurch gekennzeichnet, daß Y ein Polyethylenglykol mit einem Molekulargewicht in der Größenordnung von etwa 3000 bis 5000 und X von Methylen-4,4'-bis(phenylisocyanat) und -OR" von Ethanol abgeleitet ist.

**16.** Steifverband nach Anspruch 15, dadurch gekennzeichnet, daß Y ein Molekulargewicht von etwa 4000 aufweist.

**17.** Prepolymermischung für den Einsatz in orthopädischen Steifverbänden, enthaltend ein Polyisocyanatprepolymer und eine Menge von etwa 0,5 bis 5 Gew.-% bezogen auf das Gewicht des Prepolymeren, eines oder mehrerer hydrophiler Bisurethane, wobei deren Verbindungen aus den Formeln I und II ausgewählt sind:

R(O)CNH-X-NHC(O)O-Y-O(O)CNH-X-NHC(O)R (I)

Z-NHC(O)O-Y-O(O)CNH-Z (II)

worin Y eine hydrophyle Polymerkette mit einem Molekulargewicht in der Größenordnung von etwa 1000 bis 8000 ist, jedes X der Formel I und jedes Z der Formel II gleich oder verschieden sein kann und ausgewählt ist aus aromatischen, cycloaliphatischen oder aliphatischen Gruppen, R -OR" ist, wobei -OR" von monofunktionallen C1-C16 Alkoholen oder von Oxyalkylenaddukten monofunktioneller Alkohole der allgemeinen Formel IV:

$$R'''-O-(CH_2\overset{CH_3}{\overset{|}{C}}HO)_q(CH_2CH_2)_mH$$

(IV)

abgeleitet ist, worin R' " H oder Alkyl ist und q und m unabhängig 0 oder ganze Zahlen von 1 oder mehr sind, vorausgesetzt, daß, wenn entweder q oder m 0 ist, der jeweils andere eine ganze Zahl von 1 oder mehr ist.

## Claims

**1.** An orthopedic casting material comprising a fabric coated or impregnated with a combination of a reactive fluid polyiaocyanate prepolymer which hardens when said resin is wetted with water to form polyurethanes and which in order to reduce tack, slip and foaming contains at least one auxiliary, characterized in that in the polyiaocyanate prepolymer used for coating the fabric hydrophilic bisurethanes are used as detackifying auxiliaries in the amount of about 0.5 to 6%-wt., relative to the weight of the prepolymer, the compounds of which are selected from the formulas I and II:

R(O)CNH-X-NHC(O)O-Y-O(O)CNH-X-NHC(O)R (I)

Z-NHC(O)O-Y-O(O)CNH-Z (II)

where Y is a hydrophilic polymeric chain having a molecular weight in the range of about 1000 to 8000; each X in formula I and each Z in formula II may be the same or different and is selected from aromatic, cycloaliphatic or aliphatic groups; and R is -OR" where -OR" is derived from monofunctional $C_1$-$C_{16}$ alcohols or from oxyalkylene adducts of monofunctional alcohols of the general formula IV:

$$R''' - O - (CH_2\overset{CH_3}{\overset{|}{C}}HO)_q(CH_2CH_2)_mH$$

(IV)

where R''' is H or alkyl and Q and m are independently 0 or integers of 1 or more, provided that if one of q and m are 0, the other of q and m is an integer of 1 or more.

2. The casting material according to claim 1, characterized in that the fabric is a knit fibreglass fabric.

3. The casting material according to claim 1, characterized in that the polyiaocyanate prepolymer comprises the reaction product of diphenylmethane diisocyanate and polypropylene glycol.

4. The casting material according to claim 1, characterized in that Y is derived form diols of the general formula (III):

$$H(OCH_2CH_2)_m - (O\overset{CH_3}{\overset{|}{C}}HCH_2)_n - OR'O - (CH_2\overset{CH_3}{\overset{|}{C}}HO)_n - (CH_2CHO)_mH$$

(III)

where m = an integer of 1 or more and n = 0 or an integer of 1 or more, and R' = $-CHCH_2-$ or $-CH(CH_3)CH_2-$.

5. The casting material according to claim 4 where Y is selected from the group consisting of polyethyleneoxides, polypropyleneoxides, and random or block ethylene/propylene oxide copolymers.

6. The casting material according to claim 5 characterized in that Y is a polyethylene glycol having a molecular weight in the range of about 3000 to 5000.

7. The casting material according to claim 6, characterized in that Y is a polyethylene glycol having a molecular weight of about 4000.

8. The casting material according to claim 1, characterized in that the bisurethane has the formula I.

9. The casting material according to claim 1, characterized in that the bisurethane has the formula II.

10. The casting material according to claim 8, characterized in that X is derived from a diisocyanate selected from the group consisting of toluene diisocyanate, methylene-4,4'-bis(phenylisocyanate), methylene-4,4'bis(cyclohexyl)diisocyanate, isophorone diisocyanate, 1,4-cyclohexane diisocyanate, hexamethylene diisocyanate, and mixtures thereof.

11. The casting material according to claim 9, characterized in that X is derived from a monoisocyanate selected from the group consisting of phenyl isocyanate, cyclohexyl isocyanate, butyl isocyanate and mixtures thereof.

12. The casting material according to claim 8, characterized in that -OR" is derived from methanol or ethanol.

13. The casting material according to claim 8, characterized in that Y is a polyethylene glycol having a molecular weight in the range of about 3000 to 5000, X is derived from methylene-4,4'-bis(phenylisocyanate), and -OR" is derived from ethanol.

14. The casting material according to claim 13, characterized in that Y has a molecular weight of about 4000.

15. The casting material of claim 1, characterized in that Y is a polyethylene glycol having a molecular weight in the range of about 3000 to 5000, X is derived from methylene-4,4'-bis(phenylisocyanate), and -OR" is

derived from ethanol.

16. The casting material according to claim 15, characterized in that Y has a molecular weight of about 4000.

17. A prepolymer mixture for use in orthopedic casting material comprising polyisocyanate prepolymer and a quantity of about 0.5 to 5%-wt., relative to the weight of the prepolymer, of one or more hydrophilic bisurethanes, the compounds thereof being selected from the formulas I and II:

R(O)CNH-X-NHC(O)O-Y-O(O)CNH-X-NHC(O)R (I)

Z-NHC(O)O-Y-O(O)CNH-Z (II)

where Y is a hydrophilic polymeric chain having a molecular weight in the range of about 1000 to 8000; each X in formula I and each Z in formula II may be the same or different and is selected from aromatic, cycloaliphatic or aliphatic groups; and R is -OR" where -OR" is derived from monofunctional $C_1$-$C_{16}$ alcohols or from oxyalkylene adducts of monofunctional alcohols of the general formula IV:

$$R''' - O - (CH_2\overset{CH_3}{\overset{|}{C}}HO)_q(CH_2CH_2)_mH$$

(IV)

where R'" is H or alkyl and q and m are independently 0 or integers of 1 or more, provided that if one of q and m are 0, the other of q and m is an integer of 1 or more.

## Revendications

1. Pansement ou bandage rigide, orthopédique, contenant un produit textile plat, imprégné ou revêtu d'une combinaison d'un prépolymère à base de polyisocyanate, fluide et réactif, qui, par mouillage de la résine avec de l'eau, réagit pour se transformer en polyuréthannes sous durcissement et qui contient au moins un adjuvant en vue de la réduction de l'adhésivité, du glissement et du moussage, caractérisé en ce que des bisuréthannes hydrophiles sont incorporés, à titre d'adjuvants conférant un caractère anticollant ou antiadhésif au prépolymère de polyisocyanate utilisé en vue du revêtement de la matière textile plane, en une proportion d'environ 0,5 à 5% en poids, par rapport au poids du prépolymère, dont les composés sont choisis parmi ceux répondant aux formules I et II :

RO(O)CNH-X-NHC(O)O-Y-O(O)CNH-X-NHC(O)R (I)

Z-NHC(O)O-Y-O(O)CNH-Z (II)

dans lesquelles Y représente une chaîne polymérique hydrophile d'un poids moléculaire d'un ordre de grandeur d'environ 1000 à 8000, chaque symbole X dans la formule I et chaque symbole Z dans la formule II peuvent être identiques ou différents et sont choisis parmi des radicaux aromatiques, cycloaliphatiques ou aliphatiques, R -OR", où -OR" dérive d'alcools monohydroxylés en $C_1$ à $C_{16}$, ou d'adduits oxyalkyléniques d'alcools monofonctionnels de la formule IV :

$$R'''-O-(CH_2\overset{CH_3}{\overset{|}{C}}HO)_q(CH_2CH_2)_mH \qquad (IV)$$

dans laquelle R'" représente un atome d'hydrogène ou un radical alkyle et q et m sont indépendamment égaux à 0 ou représentent indépendamment des nombres entiers égaux ou supérieurs à 1, avec la condition que lorsque q ou m est égal à 0, l'autre indice représente à chaque fois un nombre entier égal ou supérieur à 1.

2. Pansement rigide suivant la revendication 1, caractérisé en ce que la matière textile plane est un tissu à mailles en fibres de verre.

3. Pansement rigide suivant la revendication 1, caractérisé en ce que le prépolymère à base de polyisocyanate contient le produit de la réaction du diisocyanate de diphénylméthane et du polypropylèneglycol.

4. Pansement rigide suivant la revendication 1, caractérisé en ce que Y dérive de diols de la formule générale III :

$$H(OCH_2CH_2)_m-(O\overset{CH_3}{\overset{|}{C}}HCH_2)_n-OR'O-(CH_2\overset{CH_3}{\overset{|}{C}}HO)_n-(CH_2CHO)_mH \qquad (III)$$

dans laquelle m représente un nombre entier égal ou supérieur à 1 et n est égal à 0 ou représente un nombre entier égal ou supérieur à 1 et R' représente un radical $-CHCH_2-$ ou $-CH(CH_3)CH_2-$.

5. Pansement rigide suivant la revendication 4, caractérisé en ce que Y est choisi dans le groupe formé par les polyoxydes d'éthylène, les polyoxydes de propylène et les copolymères irréguliers ou séquencés de l'oxyde d'éthylène/oxyde de propylène.

6. Pansement rigide suivant la revendication 5, caractérisé en ce que Y est un polyéthylèneglycol d'un poids moléculaire d'un ordre de grandeur d'environ 3000 à 5000.

7. Pansement rigide suivant la revendication 6, caractérisé en ce que Y est un polyéthylèneglycol d'un poids moléculaire d'environ 4000.

8. Pansement rigide suivant la revendication 1, caractérisé en ce que le bisuréthanne répond à la formule I.

9. Pansement rigide suivant la revendication 1, caractérisé en ce que le bisuréthanne répond à la formule II.

10. Pansement rigide suivant la revendication 8, caractérisé en ce que X dérive d'un diisocyanate qui est choisi dans le groupe formé par le diisocyanate de toluène, le méthylène-4,4'-bis(isocyanate de phényle), le diisocyanate de méthylène-4,4'-bis(cyclohexyle), le diisocyanate d'isophorone, le diisocyanate de 1,4-cyclohexane, le diisocyanate d'hexaméthylène et leurs mélanges.

11. Pansement rigide suivant la revendication 9, caractérisé en ce que X dérive d'un monoisocyanate, qui est choisi dans le groupe formé par l'isocyanate de phényle, l'isocyanate de cyclohexyle, l'isocyanate de butyle et leurs mélanges.

12. Pansement rigide suivant la revendication 8, caractérisé en ce que -OR" dérive du méthanol ou de l'éthanol.

13. Pansement rigide suivant la revendication 8, caractérisé en ce que Y est un polyéthylèneglycol d'un poids moléculaire d'un ordre de grandeur d'environ 3000 à 5000, X dérive du méthylène-4,4'-bis(isocyanate de phényle) et -OR" dérive de l'éthanol.

14. Pansement rigide suivant la revendication 13, caractérisé en ce que Y possède un poids moléculaire d'environ 4000.

15. Pansement rigide suivant la revendication 1, caractérisé en ce que Y est un polyéthylèneglycol d'un poids moléculaire d'un ordre de grandeur d'environ 3000 à 5000 et X dérive du méthylène-4,4'-bis(isocyanate de phényle) et -OR" dérive de l'éthanol.

16. Pansement rigide suivant la revendication 15, caractérisé en ce que Y possède un poids moléculaire d'environ 4000.

17. Mélange à base d'un prépolymère pour l'incorporation à des pansements rigides et orthopédiques, contenant un prépolymère à base de polyisocyanate et une proportion d'environ 0,5 à 5% en poids par rapport au poids du prépolymère, d'un ou de plusieurs bisuréthannes hydrophiles, où leurs composés sont choisis parmi ceux répondant aux formules I et II :

RO(O)CNH-X-NHC(O)O-Y-O(O)CNH-X-NHC(O)R (I)

Z-NHC(O)O-Y-O(O)CNH-Z (II)

dans lesquelles Y représente une chaîne polymérique hydrophile d'un poids moléculaire d'un ordre de

grandeur d'environ 1000 à 8000, chaque symbole X dans la formule I et chaque symbole Z dans la formule II peuvent être identiques ou différents et sont choisis parmi des radicaux aromatiques, cycloaliphatiques ou aliphatiques, R -OR", où -OR" dérive d'alcools monohydroxylés en $C_1$ à $C_{16}$, ou d'adduits oxyalkyléniques d'alcools monofonctionnels de la formule IV :

$$R'''-O-(CH_2\overset{\displaystyle CH_3}{\overset{|}{C}}HO)_q(CH_2CH_2)_mH \qquad (IV)$$

dans laquelle R'" représente un atome d'hydrogène ou un radical alkyle et q et m sont indépendamment égaux à 0 ou représentent indépendamment des nombres entiers égaux ou supérieurs à 1, avec la condition que lorsque q ou m est égal à 0, l'autre indice représente à chaque fois un nombre entier égal ou supérieur à 1.